# EUROPEAN PATENT SPECIFICATION

(11) **EP 1 767 565 B1**
(45) Date of publication and mention of the grant of the patent: **01.04.2015**
(21) Application number: 05758164.7
(22) Date of filing: 06.07.2005
(51) Int. Cl.: C08G 73/02, C07C 209/84, C07C 211/54, H01B 1/12, H05B 33/14, H05B 33/22

(54) **PROCESS FOR PURIFICATION OF OLIGOANILINES AND OLIGOANILINES**
VERFAHREN ZUR REINIGUNG VON OLIGOANILINEN UND OLIGOANILINE
OLIGOANILINES ET PROCÉDÉ DE PURIFICATION DESDITES OLIGOANILINES

(30) Priority: 09.07.2004 JP 2004202715
(43) Date of publication of application: 28.03.2007
(73) Proprietor: Nissan Chemical Industries, Ltd., Chiyoda-ku, Tokyo 101-0054 (JP)
(72) Inventor: KATO, T., c/o Electronic Mat. Res. Lab.,, Funabashi-shi, Chiba 2748507 (JP); YOSHIMOTO, T., c/o Electronic Mat. Res. Lab., Funabashi-shi, , Chiba 2748507 (JP)
(74) Representative: Stoner, Gerard Patrick
(86) International application number: PCT/JP2005/012454
(87) International publication number: WO 2006/006459

(56) References cited:
- EP-A1- 1 477 993
- EP-A1- 1 589 788
- WO-A1-99/28290
- WO-A1-03/071559
- WO-A1-2004/043117
- JP-A- 6 239 996
- JP-A- 10 168 446
- JP-A- 62 010 108
- JP-A- 62 010 108
- JP-A- 2000 204 158
- JP-A- 2002 151 272
- JP-A- 2005 108 828
- US-A- 5 510 532
- US-A- 5 641 859
- US-A- 5 641 859
- US-A- 6 160 177
- US-B1- 6 235 871
- MIYOKO OCHI ET AL: "Preparation of Linear Oligoaniline Derivatives using Titanium Alkoxide as Condensing Agent" BULLETIN OF THE CHEMICAL SOCIETY OF JAPAN, CHEMICAL SOCIETY OF JAPAN, TOKYO, JP, vol. 67, no. 6, 1 June 1994 (1994-06-01), pages 1749-1752, XP002098951 ISSN: 0009-2673
- ROBERT A SINGER ET AL: "A General Synthesis of End-Functionalized Oligoanilines via Palladium-Catalyzed Amination" JOURNAL OF THE AMERICAN CHEMICAL SOCIETY, AMERICAN CHEMICAL SOCIETY, NEW YORK, USA, vol. 120, no. 1, 14 January 1998 (1998-01-14), page 213/214, XP002098950 ISSN: 0002-7863
- JOSEPH P SADIGHI ET AL: "Palladium-Catalyzed Synthesis of Monodisperse, Controlled-Length, and Functionalized Oligoanilines" JOURNAL OF THE AMERICAN CHEMICAL SOCIETY, AMERICAN CHEMICAL SOCIETY, NEW YORK, USA, vol. 120, no. 20, 27 May 1998 (1998-05-27) , pages 4960-4976, XP002098949 ISSN: 0002-7863

## Description

### TECHNICAL FIELD

The present invention relates to a method for purification of an oligoaniline compound and an oligoaniline compound.

### BACKGROUND ART

The present applicant found a charge transporting varnish containing a charge transporting substance of low-molecular-weight oligoaniline compound dissolved in an organic solvent. The present applicant also found that the charge transporting varnish can be made into a charge transporting thin film suitable for an organic electroluminescence element (organic EL element for short hereinafter) which exhibits outstanding characteristic properties. (See Patent Document 1: JP-A 10-509751.)

An oligoaniline compound is subject to contamination with impurities, and a contaminated oligoaniline compound shortens the life of an organic EL element or prevents an organic EL element from exhibiting its characteristic properties invariably.

Such impurities may include trace metals, such as Ca, Fe, and Na, originating from man-caused contamination or contamination of equipments at time of manufacturing and metal reagents used to produce an oligoaniline compound.

Reducing residual metals in an oligoaniline for an organic EL element as much as possible is important in view of the recent trend for environmental consideration, social responsibility, and meticulous metal control in the field of electronic materials.

In addition, it is especially important to establish a method for industrial production of organic EL elements with uniform quality and excellent reproducibility. In order to retain the reproducibility on an industrial scale, a method which does not need complex operations is generally desirable.

The low-molecular-weight oligoaniline compound mentioned above is liable to oxidation by oxygen in air or solvent during its production or long-term storage. Upon oxidation, it assumes an oxidized form having the quinonediimine structure. Oligoaniline in oxidized form deteriorates the uniformity of film thickness and the reproducibility of film formation if it exists in an excess amount.

The present applicant proposed a method for removing oligoaniline in oxidized form by treating an oligoaniline compound with a reducing agent such as hydrazine (See Patent Document 2: WO 03/071559). The present applicant also found that a high-viscosity solvent is suitable for a varnish that gives a highly uniform thin film (See Patent Document 3: WO 04/043117), thereby performing improvement in uniformity of a thin film.

For the foregoing improved method to be applied to reproducible industrial production under easy management at the manufacturing process, it is necessary that the oligoaniline compound as the raw material should be purified so that the content of oligoaniline in oxidized form is limited to a constantly low level.

Thus, a high purity of the oligoaniline compound as the raw material is essential for the organic EL element to exhibit its stable reproducible characteristic properties. This necessitates the development of a new method for purification.

Patent Document 1:
   JP-A 10-509751
Patent Document 2:
   WO 03/071559
Patent Document 3:
   WO 04/043117

US 6,160,177 describes preparation of tetraaniline in the emeraldine oxidation state by ferric chloride-promoted oxidative coupling of N-phenyl-1,4-phenylenediamine HCl salt. The reaction mixture is recrystallized from PhMe, and treated with hydrazine to convert to tetraaniline in the leucoemeraldine oxidation state. The tetraaniline is then contacted with an oxidative coupling agent to produce oligoaniline oligomers having a particular formula.

US 6,235,871 and WO 99/28290 describe a method for the preparation of oligoanilines using transition metal-catalyzed amination of aryl halides and an orthogonal protective group scheme. Oligoaniline precursors are formed which can be converted to deprotected oligoanilines.

JP 2002-151272 describes an electroluminescent element composed of a positive and a negative electrode and one or more layers of an organic compound between them. It also has a conductive thin film formed of an oligoaniline derivative having a particular formula, an electron-accepting dopant and salt as a carrier transport layer between the positive electrode and the organic layer.

JP 2000-204158 describes an aromatic amine derivative having repeating units of two particular formulae and a specific number average molecular weight. By using the aromatic amine derivative and an electron-accepting dopant to form a salt, a soluble conductive compound can be prepared.

JP 10-168446 describes an organic electroluminescent element having at least one layer containing an oligoaniline compound having a particular formula between a pair of electrodes.

JP 2005-108828 describes an electric charge transport varnish prepared by dissolving or dispersing an electric charge transport oligomer and a non-crystalline organic acid in at least one kind of organic solvent.

Ochi, M. et al. [Bull. Chem. Soc. Jpn., 67, 1749-1752 (1994)] describes the preparation of linear oligoaniline derivatives by reacting together aromatic amines and phenols using assorted titanium alkoxides as condensing agents.

Singer, R. A. et al. [J. Am. Chem. Soc. 1998, 120, 213-214] describes palladium-catalyzed amination for the synthesis of end-functionalized oligoanilines.

Sadighi, J. P. et al. [J. Am. Chem. Soc., 1998, 120, 4960-4976] describe a method for the preparation of oligoanilines using palladium-catalyzed amination of aryl halides, together with an orthogonal protective group scheme, to form oligoaniline precursors which can be converted to deprotected oligoanilines.

JP 62-10108 describes the purification of an electroconductive polymer by recirculating an organic solvent between an adsorbing layer, comprising a specific adsorbent mixture, and an electroconductive polymer-containing tower connected therewith.

US 5,510,532 describes conductive polymers having repeating units of a particular formula prepared by chemical or electrochemical oxidation of aniline derivatives of a particular formula.

US 5,641,859 describes a polyaniline and its method of preparation. The method comprises reacting a polyaniline with strong base, and reacting it with sultone to form a side chain alkanesulfonic acid group. A precipitate is formed by doping with protonic acid, and the precipitate is dissolved in aqueous alkaline solution. Excess alkali is removed and the resulting aqueous solution is contacted with a H⁺-type ion exchange resin.

### DISCLOSURE OF INVENTION

### PROBLEMS TO BE SOLVED BY THE INVENTION

The present invention was completed in view of the foregoing. It is an object of the present invention to provide a method for efficient purification of an oligoaniline compound, the method giving a purified oligoaniline compound which has a low content of impurities and permits the organic EL element to invariably exhibit its excellent characteristic properties.

### MEANS FOR SOLVING THE PROBLEMS

To achieve the above-mentioned object, the present inventors carried out a series of researches which led to the findings that an oligoaniline compound containing oligoaniline in oxidized form in an amount exceeding a prescribed level greatly decreases in the content of oligoaniline in oxidized form and residual metals if it is dissolved in a solvent and the resulting solution is treated with a prescribed amount of activated carbon which is subsequently removed and then the solution is subjected to recrystallization. Moreover, the findings indicate that the thus purified oligoaniline compound with a low content of oligoaniline in oxidized form and residual metals can be made into a charge transporting thin film for an organic EL element which invariably exhibits its excellent characteristic properties. The present invention is based on these findings.

The present invention covers the following aspects as set out in the claims.
1. A method for purification of an oligoaniline compound which has the steps as defined in claim 1. The process includes dissolving an oliganiline compound as defined in claim 1 , which is represented by the formula (1) below, in a solvent, thereby giving a solution containing the oligoaniline compound, treating the solution with activated carbon in an amount of 4 to 20 wt% based on the amount of the oligoaniline compound, removing the activated carbon and performing recrystallization, thereby obtaining a purified oligoaniline compound represented by the formula (1) below, which has an absorption coefficient ε no higher than 30 at a wavelength of 560 nm. 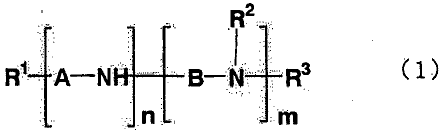 where R¹, R², and R³ independently denote hydrogen, hydroxyl group, halogen group, amino group, silanol group, thiol group, carboxyl group, sulfonic group, phosphoric group, phosphate group, ester group, thioester group, amide group, nitro group, monovalent hydrocarbon group, organooxy group, organoamino group, organosilyl group, organothio group, acyl group, or sulfonyl group; and A and B independently denote a divalent group represented by the formula (2) or (3) below. 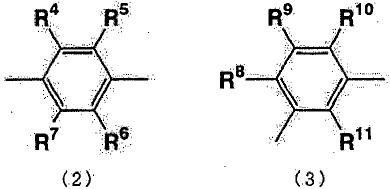 where R⁴ to R¹¹ independently denote hydrogen, hydroxyl group, halogen group, amino group, silanol group, thiol group, carboxyl group, sulfonic group, phosphoric group, phosphate group, ester group, thioester group, amide group, nitro group, monovalent hydrocarbon group, organooxy group, organoamino group, organosilyl group, organothio group, acyl group, or sulfonyl group; and m and n independently denote an integer equal to or larger than 1 such that m + n s 20.
2. The method for purification of an oligoaniline compound as defined in paragraph 1 above, wherein the purified oligoaniline compound represented by the formula (1) contains no more than 1 ppm of residual metals including Li, Mg, Ca, Fe, Cu, Zn, Ti, Sn, Na, and K.
3. The method for purification of an oligoaniline compound as defined in any of paragraphs 1 to 2 above, wherein said oligoaniline compound is one represented by the formula (4) below. 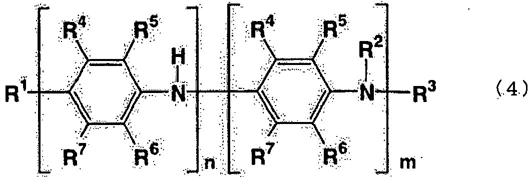 where R¹ to R⁷, and m and n are defined as above.
4. An oligoaniline compound represented by the formula (1) below which contains no more than 1 ppm of residual metals including Li, Mg, Ca, Fe, Cu, Zn, Ti, Sn, Na, and K. 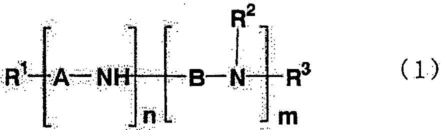 where R¹, R², R³, A, and B are as set out in claim 10.
5. An oligoaniline compound of paragraph 4 above which has an absorption coefficient ε no higher than 400 at a wavelength of 560 nm.
6. The oligoaniline compound as defined in paragraph 4 or 5 above, which is represented by the formula (4) below. 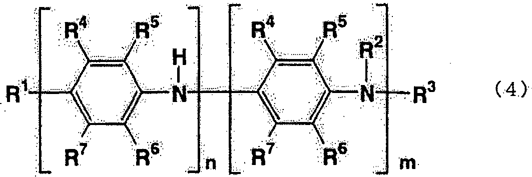 where R¹ to R⁷ and m and n are defined as in paragraph 4 above.
7. The charge transporting varnish which contains an oligoaniline compound defined in any of paragraphs 4 to 6 above.
8. The charge transporting thin film prepared from the charge transporting varnish defined in paragraph 7 above, which is characterized by an average value of surface roughness no larger than 1 nm.
9. The organic electroluminescence element which has the charge transporting thin film defined in paragraph 8 above.

### EFFECTS OF THE INVENTION

The method according to the present invention yields a purified oligoaniline compound which contains a very small amount of oligoaniline in oxidized form and residual metals. The purified oligoaniline compound can be made into a charge transporting thin film free of foreign matters, which contributes to long-lived organic EL elements and allow them to invariably exhibit their characteristic properties. Moreover, the method for purification that reduces the content of oligoaniline in oxidized form below a certain level yields oligoaniline of constant quality under easy process control.

### BRIEF DESCRIPTION OF DRAWINGS

Fig. 1 is an atomic force microphotograph in Example 9.
Fig. 2 is an atomic force microphotograph in Example 10.
Fig. 3 is an atomic force microphotograph in Comparative Example 6.
Fig. 4 shows the light emitting surface which appears when the OLED element in Example 12 is driven at 8 V.
Fig. 5 shows the light emitting surface which appears when the OLED element in Example 13 is driven at 8 V.
Fig. 6 shows the light emitting surface which appears when the OLED element in Example 14 is driven at 8 V.
Fig. 7 shows the light emitting surface which appears when the OLED element in Comparative Example 7 is driven at 8 V.

### BEST MODE FOR CARRYING OUT THE INVENTION

The invention will be described below in more detail.

According to the present invention, a first method for purification of an oligoaniline compound consists of the following steps. First, a produced, unpurified oligoaniline compound, which is represented by the formula (1), is dissolved in a solvent to give a solution containing an oligoaniline compound. Second, the resulting solution is treated with activated carbon in an amount of 4 to 20 wt% based on the amount of said oligoaniline compound. Third, the treated solution undergoes recrystallization. In this way there is obtained a purified oligoaniline compound represented by the formula (1), which has an absorption coefficient ε no higher than 30 at a wavelength of 560 nm. The combination of treatment with activated carbon and recrystallization gives a highly purified oligoaniline which is never obtained by recrystallization and treatment with activated carbon (or celite) which are performed alone.

The first method for purification according to the present invention is applied to a crude oligoaniline compound which is obtained just after production (or synthesis) or which has been stored for a certain period of time in its crude state. It usually contains a certain amount of oligoaniline in oxidized form.

The oligoaniline in oxidized form has a maximum absorption coefficient at a wavelength of 560 nm. It is characterized by the quinonediimine structure represented by the formula below. A crude oligoaniline compound immediately after production usually has an absorption coefficient ε of 80 to 100 or above at a wavelength of 560 nm. Also, a crude oligoaniline compound after storage in air at room temperature for 720 days or less usually has an absorption coefficient ε of about 400 to 1000 at a wavelength of 560 nm. 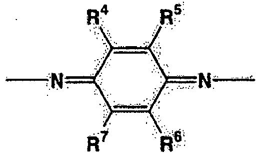 where R⁴ to R⁶ are defined as above.

A second method for purification of an oligoaniline compound according to the present invention uses the absorption coefficient ε at a wavelength of 560 nm as an index for the content of oligoaniline in oxidized form. It consists of the following steps. First, a crude oligoaniline compound, which is represented by the formula (1), is dissolved in a solvent to give a solution containing an oligoaniline compound. This crude oligoaniline compound contains oligoaniline in oxidized form and has an absorption coefficient ε of 80 to 1000 at a wavelength of 560 nm. Second, the resulting solution is treated with activated carbon in an amount of 4 to 20 wt% based on the amount of the oligoaniline compound. Third, the treated solution undergoes recrystallization. In this way there is obtained a purified oligoaniline compound represented by the formula (1), which has an absorption coefficient ε no higher than 30 at a wavelength of 560 nm.

The second method for purification is applicable to any oligoaniline compound, crude or purified by recrystallization, so long as it has an absorption coefficient ε specified above. Moreover, it is also applicable to an oligoaniline compound which has been purified by the method according to the present invention and then stored for a certain period of time so that the amount of oligoaniline in oxidized form has increased to such an extent as to give the above-mentioned absorption coefficient.

The method for purification according to the present invention may employ any solvent for dissolution and recrystallization of oligoaniline compound. The solvent is not specifically restricted so long as it dissolves an oligoaniline compound. It includes, for example, 1,4-dioxane, tetrahydrofuran, 1,3-dioxolane, diethylene glycol diethyl ether, and acetonitrile. Preferable among these examples is 1,4-dioxane.

The solvent for dissolution and recrystallization of oligoaniline compound should preferably undergo deaeration prior to use because it might oxidize the oligoaniline compound dissolved therein. Deaeration may be accomplished in any known way, such as ultrasonic deaeration and vacuum deaeration. The oxygen density (DO) in the deaerated solvent should be no more than 5%, preferably no more than 3%, and more preferably no more than 1%, although it is not specifically restricted.

The solution containing an oligoaniline compound is desirably low concentration of an oligoaniline enough for complete dissolution. An adequate concentration should be established in consideration of treatment with activated carbon and recrystallization for balanced yield and purification. Accordingly, preferable concentration is 0.1 to 10 wt%, more preferably 1 to 6 wt%.

The first and second methods for purification according to the present invention (which will be collectively referred to as the method for purification hereinafter) involve the step of treating the solution containing an oligoaniline as produced with activated carbon in an amount of 4 to 20 wt% based on the amount of the oligoaniline compound. The activated carbon is not specifically restricted in its type; however, powdery activated carbon is preferable. Treatment with activated carbon may be accomplished either by adding activated carbon to the oligoaniline-containing solution or by adding the oligoaniline-containing solution to activated carbon.

After treatment with activated carbon, the remaining activated carbon may be removed in any way. A simple way is by filtration which is carried out while the solution is hot and the oligoaniline compound remains dissolved. Filtration with a filter aid, such as celite is desirable. The amount of celite is usually 10 to 300 wt% for activated carbon.

The method for purification according to the present invention should employ an adequate amount of activated carbon. With an amount less than 4 wt%, the activated carbon does not completely remove impurities, particularly trace metals, from the oligoaniline compound. With an amount more than 20 wt%, the activated carbon hampers smooth filtration, resulting in low yields. Although the rate of removal of impurities can be increased, yields of the oligoaniline compound are lowered. This is not desirable from the view point of efficient industrial production, since yield is an especially important factor, thereby causing a problem in stable supply.

To achieve high yields while removing trace metals as much as possible, it is desirable to use the activated carbon in an amount of 4 to 15 wt% for yields higher than 90%, preferably 4 to 10 wt% for yields higher than 95%.

Incidentally, the amount of activated carbon should be based on the amount of oligoaniline compound containing impurities.

The method for purification according to the present invention involves recrystallization that follows removal of activated carbon. Prior to recrystallization, activated carbon is removed by filtration, and the resulting filtrate is freed of solvent to give a preliminarily purified oligoaniline compound, which is subsequently dissolved in a solvent again. Alternatively, by using the filtrate after removing the activated carbon by filtration, efficient recrystallization is also possible without extra operations, such as solvent removal.

The temperature for recrystallization is not specifically restricted so long as it is low enough for the dissolved oligoaniline compound to separate out. Usually, the filtrate while hot is allowed to cool to about room temperature (20°C).

After recrystallization, the oligoaniline compound which has separated out is recovered by filtration, followed by drying. The step for recovery should preferably be carried out in an atmosphere of inert gas, such as nitrogen and the subsequent step of drying should preferably be carried out under reduced pressure by using a vacuum dryer or the like, because the oligoaniline compound is liable to oxidation by oxygen in air. Usually, drying is carried out at 20°C to 200°C for 1 to 48 hours.

The method for purification according to the present invention permits treatment with activated carbon and recrystallization to be repeated several times as separate steps or consecutive steps. These two consecutive steps carried out once give an oligoaniline compound represented by the formula (1) which has an absorption coefficient ε no higher than 30 at a wavelength of 560 nm.

The thus purified oligoaniline compound, which contains a reduced amount of oligoaniline in oxidized form, can be readily formed into a charge transporting thin film.

In order to use a charge transporting thin film made from the oligoaniline compound for electronic devices, it is desirable that the content of residual metals should be decreased to not higher than 1 ppm. The method for purification according to the present invention gives a purified oligoaniline compound in which the content of residual metals, such as Li, Mg, Ca, Fe, Cu, Zn, Ti, Sn, Na, and K, is lower than 1 ppm.

The following is concerned with the oligoaniline compound to be purified by the method according to the present invention, which is represented by the formula (1).

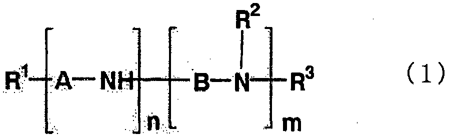

In the formula (1), R¹, R², and R³ independently denote hydrogen, hydroxyl group, halogen group, amino group, silanol group, thiol group, carboxyl group, sulfonic group, phosphoric group, phosphate group, ester group, thioester group, amide group, nitro group, monovalent hydrocarbon group, organooxy group, organoamino group, organosilyl group, organothio group, acyl group, or sulfonyl group; and A and B independently denote a divalent group represented by the formula (2) or (3) below. 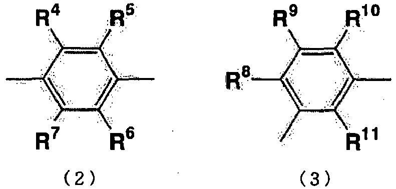 where R⁴ to R¹¹ independently denote hydrogen, hydroxyl group, halogen group, amino group, silanol group, thiol group, carboxyl group, sulfonic group, phosphoric group, phosphate group, ester group, thioester group, amide group, nitro group, monovalent hydrocarbon group, organooxy group, organoamino group, organosilyl group, organothio group, acyl group, or sulfonyl group; and m and n independently denote an integer equal to or larger than 1 such that m + n s 20.

Examples of the monovalent hydrocarbon group include alkyl groups, such as methyl, ethyl, propyl, butyl, t-butyl, hexyl, octyl, and decyl groups, cycloalkyl groups, such as cyclopentyl and cyclohexyl groups, bicycloalkyl groups, such as bicyclohexyl group, alkenyl groups, such as vinyl, 1-propenyl, 2-propenyl, isopropenyl, 1-methyl-2-propenyl, 1-, 2-, or 3-butenyl, and hexenyl groups, aryl groups, such as phenyl, xylyl, tolyl, biphenyl, and naphthyl groups, aralkyl groups, such as benzyl, phenylethyl, and phenylcylohexyl groups, and derivatives thereof, with hydrogen atoms entirely or partly replaced by halogen atoms, hydroxyl groups, alkoxyl groups, or the like.

Examples of the organoxy group include alkoxy groups, alkenyloxy groups, and aryloxy groups. Their alkyl, alkenyl, and aryl groups include those enumerated above.

Examples of the organoamino group include phenylamino groups, alkylamino groups, such as methylamino, ethylamino, proylamino, butylamino, pentylamino, hexylamino, heptylamino, octylamino, nonylamino, decylamino, and laurylamino groups, dialkylamino groups, such as dimethylamino, diethylamino, dipropylamino, dibutylamino, dipentylamino, dihexylamino, diheptylamino, dioctylamino, dinonylamino, and didecylamino groups, cyclohexylamino groups, and morpholino groups.

Examples of the organosilyl compound include trimethylsilyl group, triethylsilyl group, tripropylsily group, tributylsilyl group, tripentylsilyl group, trihexylsilyl group, pentyldimethylsilyl group, hexyldimethylsilyl group, octyldimethylsilyl group, and decyldimethylsilyl group.

Examples of the organothio group include alkylthio groups, such as methylthio, ethylthio, propylthio, butylthio, pentylthio, hexylthio, heptylthio, octylthio, nonylthio, decylthio, and laurylthio groups.

Examples of the acyl group include formyl group, acetyl group, propionyl group, butyryl group, isobutyryl group, valeryl group, isovaleryl group, and benzoyl groups.

The carbon number in the alkyl, alkoxy, thioalkyl, alkylamino, organosiloxy, and organosilyl groups is not specifically restricted; however, it is usually 1 to 20, preferably 1 to 8.

Examples of the preferred substituent group include fluorine, sulfonic group, substituted or unsubstituted organooxy group, alkyl group, and organosilyl group.

The oligoaniline compound to be purified according to the methods of present invention should preferably be the one represented by the formula (4) below which has the expanded π conjugated system so that it gives a charge transporting thin film with improved charge transporting properties. The term "charge transporting properties" is synonymous with conductivity. Here, charge implies holes, electrons, and both of holes and electrons. The charge transporting properties may be possessed by the charge transporting varnish prepared from the oligoaniline compound according to the present invention or by the thin film prepared from the varnish. 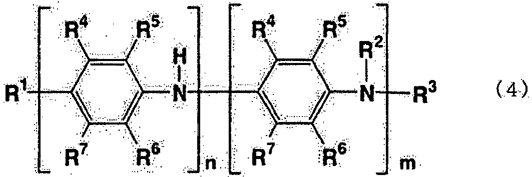 where R¹ to R⁷, and m and n are defined as above.

In the formula (4), R¹ and R² should preferably be any one selected from hydrogen atom, alkyl group of 1 to 20 carbons, particularly 1 to 4 carbons, phenyl group which may have a substituent alkyl or alkoxyl group of 1 to 4 carbons, respectively, cyclohexyl group, cyclopentyl group, biphenyl group, bicyclohexyl group, phenylcyclohexyl group, and acyl group of 2 to 4 carbons. R³ should preferably be any one selected from hydrogen atom, alkyl group of 1 to 4 carbons, and phenyl group which may have a substituent alkoxyl group.

The oligoaniline compound in which R¹ is a hydrogen atom and R³ is a phenyl group is particularly preferable. In other words, an oligoaniline compound having both ends blocked with phenyl groups is preferable. Compounds with these substituents are claimed in claims 10-12.

The substituent groups, R⁴ to R¹¹, should preferably be any one selected from hydrogen atom, alkyl group, alkoxyl group, alkoxyalkyl group, alkenyl group, acyl group, sulfonic group, hydroxyl group, phenyl group which may have a substituent alkyl or alkoxyl group of 1 to 4 carbons, respectively, cyclohexyl group, cyclopentyl group, biphenyl group, bicyclohexyl group, and phenylcyclohexyl group.

Particularly preferable examples of R⁴ to R¹¹ include a hydrogen atom, alkyl group of 1 to 20 carbons, alkoxyl group of 1 to 20 carbons, alkoxyalkyl group composed of an alkoxyl group of 1 to 20 carbons and an alkyl group of 1 to 20 carbons, alkenyl group of 2 to 4 carbons, acyl group of 2 to 4 carbons, benzoyl group, sulfonic group, hydroxyl group, phenyl group which may have a substituent alkyl or alkoxyl group of 1 to 4 carbons, respectively, cyclohexyl group, cyclopentyl group, biphenyl group, bicyclohexyl group, and phenylcyclohexyl group. Most desirable among these examples are a hydrogen atom, alkyl group of 1 to 4 carbons, alkoxyl group of 1 to 4 carbons, alkoxyalkyl group composed of an alkoxyl group of 1 to 4 carbons and an alkyl group of 1 to 4 carbons, vinyl group, 2-propenyl group, acetyl group, benzoyl group, sulfonic group, hydroxyl group, phenyl group which may have a substituent alkyl of 1 to 4 carbons or alkoxyl group of 1 to 4 carbons, cyclohexyl group, biphenyl group, bicyclohexyl group, and phenylcyclohexyl group.

Incidentally, the two benzene rings in the formula (4) may have the identical or different substituent groups carrying the same symbols.

The oligoaniline compound according to the present invention should preferably be without molecular weight distribution, in other words, monodisperse oligoaniline compound in consideration of high solubility and uniform charge transporting properties. It should normally have a molecular weight no smaller than 200, preferably no smaller than 400 for low volatility and good charge transporting properties, and no larger than 5000, preferably no larger than 2000 for high solubility.

In the general formulas (1) and (4), the sum of m+n should preferably be no smaller than 4 for good charge transporting properties, and more preferably no larger than 16 for good solubility in solvent.

Those compounds meeting this requirement include phenyltetraaniline and phenylpentaaniline, which are soluble in organic solvent.

The above-mentioned oligoaniline compounds may be synthesized by any method without specific restrictions. Typical methods are disclosed in Bulletin of Chemical Society of Japan, 1994, vol. 67, p. 1749 to 1752, and Synthetic Metals, U.S., 1997, vol. 84, p. 119 to 120.

The charge transporting varnish according to the present invention contains an oligoaniline compound with no more than 1 ppm trace amounts of residual metals, such as Li, Mg, Ca, Fe, Cu, Zn, Ti, Sn, Na, and K.

The oligoaniline compound should contain as little residual metals as possible if it is to be used for the charge transporting varnish as an electronic material. It should also contain as little oligoaniline in oxidized form as possible. Otherwise, the varnish prepared from it gives a charge transporting thin film with a large average surface roughness (Ra). Such a film, when applied to an organic EL element, may prevent uniform light emission.

For the charge transporting varnish according to the present invention to give a flat thin film free of foreign matters for uniform light emission from an organic EL element, the purified oligoaniline compound should have an absorption coefficient ε no higher than 400, preferably no higher than 250, more preferably no higher than 100, and most desirably no higher than 30, at a wavelength of 560 nm.

The charge transporting varnish may be prepared by using any solvent which dissolves or decomposes the oligoaniline compound. Examples of the solvent include cyclohexanol, ethylene glycol, ethylene glycol diglycidyl ether, 1,3-octylene glycol, diethylene glycol, dipropylene glycol, triethylene glycol, tripropylene glycol, 1,3-butanediol, 1,4-butanediol, propylene glycol, hexylene glycol, N,N-dimethylformamide, N,N-dimethylacetamide, N-methylpyrrolidone, N-methylformanilide, N,N'-dimethylimidazolidinone, dimethylsulfoxide, chloroform, toluene, and methanol. They may be used alone or in combination with one another.

The charge transporting varnish may be incorporated with an adequate amount of charge transporting substance, such as electron accepting dopant and hole transporting dopant. A desirable charge transporting substance for the present invention is a sulfonic acid derivative represented by the formula (5) below. Examples of the sulfonic acid derivative include sulfosalicylic acid derivatives, such as 5-sulfosalicylic acid. 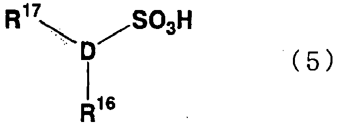 where D denotes a benzene ring, naphthalene ring, anthracene ring, phenanthrene ring, or heterocyclic ring; and R¹⁶ and R¹⁷ independently denote a carboxyl group or hydroxyl group.

The charge transporting thin film according to the present invention is produced from the charge transporting varnish mentioned above, and it has an average surface roughness Ra no larger than 1 nm.

With a value of Ra exceeding 1 nm, the charge transporting thin film has a low light emitting efficiency. As a result, it is highly possible that the light emitting surface may lack uniformity in the organic EL element.

This thin film can be formed by applying the charge transporting varnish onto a substrate, followed by solvent evaporation. The method for varnish application is not specifically restricted; it includes dipping, spin coating, roller transfer, roll coating, ink jet printing, spraying, and brushing.

Solvent evaporation may be accomplished in an adequate atmosphere by using a hot plate or oven. The temperature may be 40 to 250°C, which is high enough for solvent evaporation.

The charge transporting thin film is not specifically restricted in thickness. It usually has a thickness of 5 to 200 nm if it is to be used as the charge injection layer in the organic EL element.

The charge transporting thin film mentioned above will find use as a thin film constituting an organic EL element.

To be concrete, it may be used as a charge injection layer in an organic EL element composed of organic thin layers such as electron transporting layer, light emitting layer, hole transporting layer, and charge injection layer, which are held between a cathode and an anode.

Any known materials may be used for the cathode, anode, electron transporting layer, hole transporting layer, and light emitting layer, which constitute an organic EL element.

### EXAMPLES

The invention will be described in more detail with reference to the following Examples and Comparative Examples, which are not intended to restrict the scope thereof.

### [Synthesis Example 1]

Phenyltetraaniline (PTA for short hereinafter) was synthesized from p-hydroxydiphenylamine and p-phenylenediamine according to the method described in Bulletin of Chemical Society of Japan, 1994, vol. 67, p. 1749 to 1752. (Light blue solid, 85% yields)

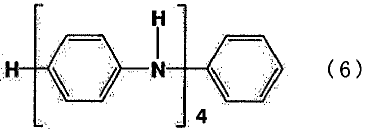

### [1] Purification of oligoaniline compound

### [Example 1]

A one liter three-neck round-bottom flask was charged in a nitrogen atmosphere with 20 g (0.0452 mmol) of PTA (obtained in Synthesis Example 1), 2.0 g (10 wt% of PTA) of activated carbon (from JUNSEI CHEMICAL CO., LTD), and 500 g of dehydrated, ultrasonically deaerated 1,4-dioxane (from KANTO CHEMICAL CO., INC).

The flask was heated with stirring in an oil bath for 1 hour with the temperature inside kept at 90°C so that PTA was dissolved completely. The contents in the flask, mixed with 50 g of celite (Celite 545 from JUNSEI CHEMICAL CO., LTD) as a filter aid, were filtered through Kiriyama glass (S-60) and Kiriyama filter paper (3C), with the temperature kept at 90°C by means of a water circulating apparatus equipped with a temperature controller, and the activated carbon was removed.

The filtrate was allowed to cool to 20°C. The resulting pale purple solution containing PTA that had separated out was transferred into a reaction vessel, which was subsequently placed in a glove box. The relative humidity in the glove box was reduced to 5% by flowing nitrogen. PTA was filtered off through a Buchner funnel in the glove box, with the relative humidity therein kept at 5%. PTA remaining on the Buchner funnel was washed sequentially with 200 mL of 1,4-dioxane, 200 mL of dehydrated toluene, and 200 mL of diethyl ether. The washed PTA was transferred to a 100-mL round-bottom flask by using a microspatula of fluoroplastic resin in the glove box. Nitrogen was purged from the flask by evacuation through a three-way cock attached thereto.

The PTA was dried under reduced pressure for 24 hours in a vacuum drier kept at 120°C. Thus there was obtained 19.34 g of white solid PTA (yields: 96.7%).

Incidentally, the oxygen density (DO) in the dehydrated, ultrasonically deaerated 1,4-dioxane was less than 1%, measured by using a fluorescent oxygen meter [(FO-960, with a standard sensor WPH-130) Automatic System Research Co., Ltd]. The oxygen meter was calibrated so that DO in nitrogen is 0% and DO in air is 20.9%.

The ultrasonically deaerated solvent used in the following examples has a DO less than 1%.

### [Comparative Example 1]

A one liter three-neck round-bottom flask was charged in a nitrogen atmosphere with 20 g (0.0452 mmol) of PTA obtained in Synthesis Example 1 and 500 g of dehydrated, ultrasonically deaerated 1,4-dioxane.

The flask was heated with stirring in an oil bath for 1 hour with the temperature inside kept at 90°C so that PTA was dissolved completely. The contents in the flask, mixed with 50 g of celite ("Celite 545") as a filter aid, were filtered through Kiriyama glass (S-60) and Kiriyama filter paper (3C), with the temperature kept at 90°C by means of a water circulating apparatus equipped with a temperature controller.

The filtrate was allowed to cool to 20°C. The resulting pale purple solution containing PTA that had separated out was transferred into a reaction vessel, which was subsequently placed in a glove box. The relative humidity in the glove box was reduced to 5% by flowing nitrogen. PTA was filtered off through a Buchner funnel in the glove box, with the relative humidity therein kept at 5%. PTA remaining on the Buchner funnel was washed sequentially with 200 mL of 1,4-dioxane, 200 mL of dehydrated toluene, and 200 mL of diethyl ether. The washed PTA was transferred to a 100-mL round-bottom flask by using a microspatula of fluoroplastic resin in the glove box. Nitrogen was purged from the flask by evacuation through a three-way cock attached thereto. The PTA was dried under reduced pressure for 24 hours in a vacuum drier kept at 120°C. Thus there was obtained 19.44 g of white solid PTA (yields : 97.2%).

### [Comparative Example 2]

A one liter three-neck round-bottom flask was charged in a nitrogen atmosphere with 20 g (0.0452 mmol) of PTA obtained in Synthesis Example 1, 2.0 g (10 wt% of PTA) of activated carbon, and 500 g of dehydrated, ultrasonically deaerated 1,4-dioxane.

The flask was heated with stirring in an oil bath for 1 hour with the temperature inside kept at 90°C so that PTA was dissolved completely. The contents in the flask, mixed with 50 g of celite ("Celite 545") as a filter aid, were filtered through Kiriyama glass (S-60) and Kiriyama filter paper (3C), with the temperature kept at 90°C by means of a water circulating apparatus equipped with a temperature controller.

The filtrate was allowed to cool to 20°C. The resulting pale purple solution containing PTA that had separated out was transferred into a reaction vessel, which was subsequently placed in a glove box. The relative humidity in the glove box was reduced to 5% by flowing nitrogen. PTA was filtered off through a Buchner funnel in the glove box, with the relative humidity therein kept at 5%. PTA remaining on the Buchner funnel was washed sequentially with 200 mL of 1,4-dioxane, 200 mL of dehydrated toluene, and 200 mL of diethyl ether. The washed PTA was transferred to a 100-mL round-bottom flask by using a microspatula of fluoroplastic resin in the glove box. Nitrogen was purged from the flask by evacuation through a three-way cock attached thereto. The PTA was dried under reduced pressure for 24 hours in a vacuum drier kept at 120°C. Thus there was obtained 19.08 g of white solid PTA (yields : 95.4%).

The navy filtrate was transferred to a one liter round-bottom flask in the glove box. Nitrogen was purged from the flask by evacuation through a three-way cock attached thereto. The filtrate was exposed to the atmospheric air and completely freed of solvent by means of an evaporator.

The resulting bluish PTA remaining was washed with 200 mL of diethyl ether in the glove box. The washed PTA was transferred to a 100-mL round-bottom flask by using a microspatula of fluoroplastic resin in the glove box. Nitrogen was purged from the flask by evacuation through a three-way cock attached thereto. The PTA was dried under reduced pressure for 24 hours in a vacuum drier kept at 120°C. Thus there was obtained 0.82 g of bluish solid PTA (yields: 4.1%).

The white solid PTA that had separated out in the solvent was uniformly mixed with the bluish solid PTA recovered from the filtrate, and the resulting PTA mixture was used to confirm the effect that is produced only by treatment with activated carbon.

### [Comparative Example 3]

A one liter three-neck round-bottom flask was charged in a nitrogen atmosphere with 20 g (0.0452 mmol) of PTA obtained in Synthesis Example 1 and 500 g of dehydrated, ultrasonically deaerated 1,4-dioxane.

The flask was heated with stirring in an oil bath for 1 hour with the temperature inside kept at 90°C so that PTA was dissolved completely. The resulting solution was allowed to cool to 20°C. The purple solution containing PTA that had separated out was transferred into a reaction vessel, which was subsequently placed in a glove box. The relative humidity in the glove box was reduced to 5% by flowing nitrogen. PTA was filtered off through a Buchner funnel in the glove box, with the relative humidity therein kept at 5%. PTA remaining on the Buchner funnel was washed sequentially with 200 mL of 1,4-dioxane, 200 mL of dehydrated toluene, and 200 mL of diethyl ether. The washed PTA was transferred to a 100-mL round-bottom flask by using a microspatula of fluoroplastic resin in the glove box. Nitrogen was purged from the flask by evacuation through a three-way cock attached thereto. The PTA was dried under reduced pressure for 24 hours in a vacuum drier kept at 120°C. Thus there was obtained 19.58 g of white solid PTA (yields: 97.9%).

### (1) Measurement of absorption coefficient ε at a wavelength of 560 nm

PTA has an absorption maximum due to aromatic rings in the neighborhood of 320 nm, and PTA in oxidized form has an absorption maximum due to quinonediimine structure in the neighborhood of 560 nm. It follows, therefore, that the content of oxidized form is proportional to the absorption coefficient ε in the neighborhood of 560 nm. The absorption coefficient ε is inherent in substance and hence it gives a clue to reliable quantitative determination. The PTA samples obtained in Example 1 and Comparative Examples 1 to 3 were tested for the content of their oxidized form by determining the absorption coefficient ε from the absorption spectra of ultraviolet and visible light. Incidentally, a standard length of time required to prepare solutions and perform measurement was established to prevent PTA from being oxidized at different rates in solutions thereby ensuring accurate ε values. The measurement of spectra was carried out by using an ultraviolet-visual light absorptiometer (UV-3100PC, made by SHIMADZU CORPORATION).

Solutions were prepared as follows from a sample of fresh crude PTA for control obtained in Synthesis Example 1 and samples of purified PTA obtained in Example 1 and Comparative Examples 1 to 3. Each sample, weighing 0.0028 g (6.326 µmol), was placed in a brown 100-mL volumetric flask and completely dissolved in deaerated acetonitrile for high-performance liquid chromatography (having a purity higher than 99.8%, from KANTO CHEMICAL CO., INC). The volumetric flask was filled to the mark (The resulting solution had a concentration of 6.3260 × 10⁻⁵ mol/L). The foregoing steps were carried out in five minutes for every sample.

The filled volumetric flask was shaken for two minutes to ensure complete dissolution. Three minutes later, the liquid level was corrected. One minute later, the PTA solution was examined for UV-VIS spectrum by using a quartz cell with a solution thickness of 1 cm.

Table 1 below shows the absorbance A due to PTA in oxidized form that was observed at the absorption maximum wavelength (λₘₐₓ) which is about 560 nm. Table 1 also shows the absorption coefficient ε calculated from the absorbance A according to Lambert-Beer's equation (A = ε × c x l, where c denotes the thickness of solution [cm] and 1 denotes the concentration of solution [mol/L]).

**Table 1**

| | λₘₐₓ (nm) | Concentration of solution (10⁻⁵ mol/L) | Absorbance A | Absorption coefficient ε |
|---|---|---|---|---|
| Example 1 | 560 | 6.326 | 0.0018 | 28.4540 |
| Comparative Example 1 | 560 | 6.326 | 0.0061 | 96.4274 |
| Comparative Example 2 | 560 | 6.326 | 0.0076 | 1097.0598 |
| Comparative Example 3 | 560 | 6.326 | 0.0065 | 102.7506 |
| Control | 560 | 6.326 | 0.0083 | 131.2046 |

It is noted from Table 1 that the sample in Example 1 which underwent treatment with activated carbon and recrystallization has a much lower absorption coefficient ε than the samples in Comparative Examples 1 to 3 and control. This indicates a significant decrease in the content of PTA in oxidized form in the sample of Example 1. By contrast, it is apparent that PTA in oxidized form remains in comparatively large quantities in the sample of Comparative Example 1 which underwent recrystallization only and the sample of Comparative Example 3 which underwent treatment with celite only.

It is also noted that the sample in Comparative Example 2 which underwent treatment with activated carbon only has a significantly high absorption coefficient ε. A probable reason for this is that concentration of the solvent increases the content of PTA in oxidized form. Incidentally, the PTA obtained from the filtrate gave a very high absorption coefficient ε, 5556.4338, which was calculated from the absorbance measured in the same way as mentioned above. This suggests that PTA cannot be purified by treatment with activated carbon or by concentration of the filtrate.

### (2) Analysis of trace metals

A sample of fresh crude PTA (for control) obtained in Synthesis Example 1 and samples of purified PTA obtained in Example 1 and Comparative Examples 1 to 3 were analyzed as follows for trace metals contained therein, such as Li, Mg, Ca, Fe, Cu, Zn, Ti, Sn, Na, and K. The results are shown in Table 2.

Each sample (200 mg PTA) was decomposed by microwave in the presence of 3 mL of nitric acid and 1 mL of sulfuric acid. The decomposed product was diluted 100 times, and the resulting solution was analyzed by inductively coupled plasma spectrometry with an ICP apparatus ("Vista-Pro" from Seiko Instruments Inc.).

**Table 2**

| | Metal content (ppm) | | | | | | | | | |
|---|---|---|---|---|---|---|---|---|---|---|
| | Li | Mg | Ca | Fe | Cu | Zn | Ti | Sn | Na | K |
| Example 1 | <1 | <1 | <1 | <1 | <1 | <1 | <1 | <1 | <1 | <1 |
| Comparative Example 1 | <1 | <1 | <1 | <1 | <1 | <1 | 32 | <1 | <1 | <1 |
| Comparative Example 2 | <1 | <1 | <1 | <1 | <1 | <1 | <1 | <1 | <1 | <1 |
| Comparative Example 3 | <1 | <1 | <1 | <1 | <1 | <1 | 41 | <1 | <1 | <1 |
| Control | <1 | <1 | 2.7 | 1.7 | <1 | <1 | 56 | <1 | 2 | <1 |

It is noted from Table 2 that the control sample of fresh PTA obtained immediately after synthesis contains Ca, Fe, and Na presumably originating from the apparatus or contaminants, and a relatively large amount of Ti originating from titanium alkoxide added as a catalyst.

It is also noted from Table 2 that the sample of PTA in Example 1 which underwent treatment with activated carbon and recrystallization decreased in the content of all trace metals, such as Li, Mg, Ca, Fe, Cu, Zn, Ti, Sn, Na, and K, below 1 ppm.

It is also noted from Table 2 that the sample of PTA in Comparative Example 1 which underwent recrystallization only and the sample of PTA in Comparative Example 3 which underwent treatment with celite only contain residual Ti.

It is also noted from Table 2 that the sample of PTA in Comparative Example 2 which underwent treatment with activated carbon only decreased in the content of trace metals below 1 ppm as in the case of the sample in Example 1. This suggests that treatment with activated carbon is effective in eliminating trace metals.

It is concluded from the foregoing that the combination of treatment with activated carbon and recrystallization is the simple and effective way for removal of both trace metals and PTA in oxidized form.

### [Examples 2 to 7 and Comparative Examples 4 and 5]

The same procedure as in Example 1 was repeated except that the amount of activated carbon (based on PTA) was changed as follows:
0.2 g or 1 wt% in Comparative Example 4
0.4 g or 2 wt% in Comparative Example 5
0.8 g or 4 wt% in Example 2
1.2 g or 6 wt% in Example 3
1.6 g or 8 wt% in Example 4
2.0 g or 10 wt% in Example 5
3.0 g or 15 wt% in Example 6
4.0 g or 20 wt% in Example 7

Table 3 below shows the amount and yield of PTA recovered in Examples 2 to 7 and Comparative Examples 4 and 5. All the samples of PTA obtained in these examples were white solids.

The samples obtained in these examples were analyzed for trace metals (mentioned above). The results are also shown in Table 3.

**Table 3**

| | Activated carbon (wt%) | Amount recovered (g) | Yields (%) | Metal content (ppm) | | | | | | | | | |
|---|---|---|---|---|---|---|---|---|---|---|---|---|---|
| | | | | Li | Mg | Ca | Fe | Cu | Zn | Ti | Sn | Na | K |
| Comparative Example 4 | 1 | 19.42 | 97.1 | <1 | <1 | <1 | <1 | <1 | <1 | 6 | <1 | <1 | <1 |
| Comparative Example 5 | 2 | 19.44 | 97.2 | <1 | <1 | <1 | <1 | <1 | <1 | 2 | <1 | <1 | <1 |
| Example 2 | 4 | 19.22 | 96.1 | <1 | <1 | <1 | <1 | <1 | <1 | <1 | <1 | <1 | <1 |
| Example 3 | 6 | 19.42 | 97.1 | <1 | <1 | <1 | <1 | <1 | <1 | <1 | <1 | <1 | <1 |
| Example 4 | 8 | 19.33 | 96.7 | <1 | <1 | <1 | <1 | <1 | <1 | <1 | <1 | <1 | <1 |
| Example 5 | 10 | 19.33 | 96.7 | <1 | <1 | <1 | <1 | <1 | <1 | <1 | <1 | <1 | <1 |
| Example 6 | 15 | 18.03 | 90.2 | <1 | <1 | <1 | <1 | <1 | <1 | <1 | <1 | <1 | <1 |
| Example 7 | 20 | 16.27 | 81.4 | <1 | <1 | <1 | <1 | <1 | <1 | <1 | <1 | <1 | <1 |

It is noted from Table 3 that treatment with activated carbon in an amount less than 4 wt% based on PTA is not enough to remove residual Ti completely in Comparative Examples 4 and 5.

It is also noted from Table 3 that treatment with activated carbon in an amount more than 4 wt% reduces the content of Ti below 1 ppm in Examples 2 to 7. It is also noted that activated carbon in excess of 15 wt% decreases the yields of PTA in Examples 6 and 7.

### [Example 8]

To investigate the effect of storage on purity, the sample of PTA obtained in Example 1 was stored at 23°C and 45% RH for 720 days and then purified again in the same way as in Example 1. Thus there was obtained purified 19.30 g of PTA in white solid form (yields: 96.5%). The samples of stored PTA (as control) and purified PTA were dissolved in solvent and the resulting solutions were examined for UV-VIS spectra. Table 4 below shows the absorption coefficient ε due to PTA in oxidized form that was observed at a wavelength of about 560 nm for absorption maximum.

**Table 4**

| | λₘₐₓ (nm) | Absorption coefficient ε |
|---|---|---|
| Example 8 | 560 | 27.8090 |
| Control | 560 | 409.4214 |

It is noted from Table 4 that the sample of PTA as control which had been purified in the same way as in Example 1 and then stored for 720 days under the condition mentioned above gave an absorption coefficient ε as high as 409.4214. This suggests that the sample contains a large amount of PTA in oxidized form that resulted from oxidation by air during storage. It is also noted from Table 4 that the sample of PTA which had been stored and then purified by treatment with activated carbon and recrystallization gave an absorption coefficient ε of 27.8090, which is close to the one observed in Example 1. This suggests that the method for purification according to the present invention can recover high-purity PTA from impure PTA containing PTA in oxidized form.

### [2] Charge transporting varnish and charge transporting thin film

### [Example 9]

A charge transporting varnish (containing 4.2% solids) was prepared by completely dissolving the sample of PTA (0.0500 g or 0.1130 mmol) purified in Example 1 and 5-sulfosalycilic acid (5-SSA) (0.0986 g or 0.4520 mmol) represented by the formula (7) below in N,N-dimethylacetamide (DMAc) (0.8757 g) in an atmosphere of nitrogen and then incorporating the resulting solution with cyclohexanol (c-HexOH) (2.6270 g).

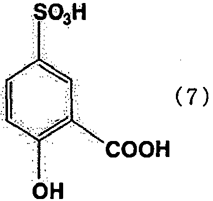

### [Example 10]

To investigate the effect of storage on purity, the sample of PTA obtained in Example 1 was stored at 23°C and 45% RH for 370 days and then purified again in the same way as in Example 1. The sample of stored PTA was dissolved in solvent and the resulting solution was examined for UV-VIS spectra. It was found from the spectra that the absorption coefficient ε due to PTA in oxidized form at a wavelength of about 560 nm for absorption maximum is as high as 211.8242. This suggests a high content of PTA in oxidized form. Incidentally, the sample of stored PTA differs from the sample of fresh PTA in Example 1 only in the content of PTA in oxidized form; in other words, the former contains the same trace metals in the same amount as the latter because the former has already undergone purification by treatment with activated carbon, filtration with the aid of celite, and recrystallization.

The sample of stored PTA was made into the charge transporting varnish in the same way as in Example 9.

### [Example 11]

The procedure of Example 9 was repeated to prepare a charge transporting varnish from the sample of PTA purified in Example 8.

### [Comparative Example 6]

The procedure of Example 9 was repeated to prepare a charge transporting varnish from the sample of PTA which was prepared in Example 8 and stored for 720 days for purification. It was found that the absorption coefficient ε due to PTA in oxidized form at a wavelength of about 560 nm for absorption maximum is 409.4214 as mentioned above. The sample of stored PTA differs from the sample of fresh PTA in Example 1 only in the content of PTA in oxidized form; in other words, the former contains the same trace metals in the same amount as the latter because the former has already undergone purification by treatment with activated carbon, filtration with celite, and recrystallization.

A hole transporting thin film was formed from each of the charge transporting varnishes prepared in Examples 9 to 11 and Comparative Example 6 by spin coating onto an ITO-coated glass substrate.

The resulting thin film was observed under an atomic force microscope (AFM, nanoscope Type IV, dimension 3100, from Digital Instruments, Veeco Instruments). It was also examined for average surface roughness (Ra) within an area of 5 by 5 µm. Observation under an AFM was carried out according to Tapping method with a scanning rate of 1 Hz and a z-range of 100 nm.

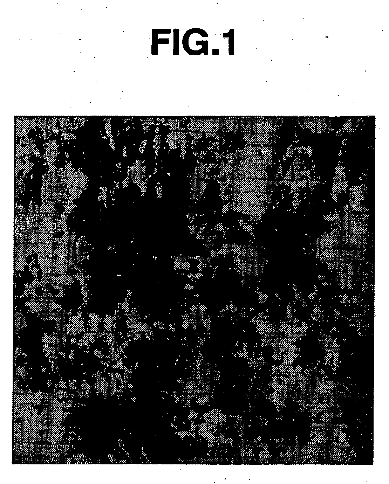

Figs. 1 to 3 show respectively the AFM diagrams pertaining to Example 9, Example 10, and Comparative Example 6. Table 5 shows respectively the absorption coefficient ε and the Ra values of the samples pertaining to Examples 9 to 11 and Comparative Example 6.

**Table 5**

| | Absorption coefficient ε | Ra (nm) |
|---|---|---|
| Example 9 | 28.4540 | 0.270 |
| Example 10 | 211.8242 | 0.286 |
| Example 11 | 27.8090 | 0.261 |
| Comparative Example 6 | 409.4214 | 6.302 |

It is noted from Table 5 that the absorption coefficient ε at a wavelength of 560 nm changed as follows after storage owing to PTA in oxidized form. The sample without storage has an initial value of 28.4540. The sample after storage for 370 days has a value of 211.8242. The sample after storage for 720 days has a value of 409.4214. This change is due to oxidation in air that takes place with the lapse of time.

It is also noted that the charge transporting thin film in Comparative Example 6 has an Ra value of 6.302 nm, which is about 20 times as rough as the Ra value in Examples 9 and 10. This is because it was formed from the charge transporting varnish containing a large amount of PTA in oxidized form as indicated by the high value of absorption coefficient ε (409.4214).

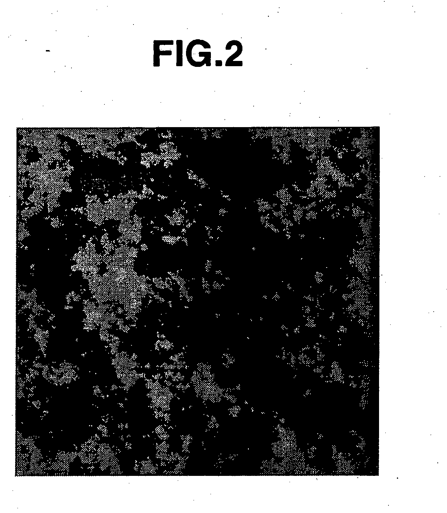

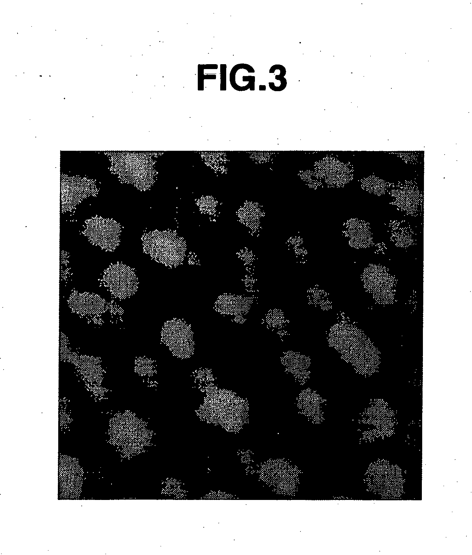

Fig. 3 showing the thin film in Comparative Example 6 differs from Figs. 1 and 2 in that there are island-like local foreign matters (about 1 µm in size) on the surface of the charge transporting thin film. This result indicates that PTA in oxidized form greatly aggravates the film forming properties when its content exceeds a certain level.

### [3] Organic EL element

### [Example 12]

An OLED element was prepared in the following manner. The charge transporting varnish prepared in Example 9 was applied by spin coating onto an ITO-coated glass substrate, so that a hole transporting thin film (30 nm thick) was formed. The substrate having a thin film formed thereon underwent vacuum deposition sequentially with α-NPD (40 nm thick), Alq₃ (60 nm thick), LiF (0.5 nm thick), and Al (100 nm thick) in a vacuum deposition apparatus. Each step of vacuum deposition was carried out at a pressure lower than 8 × 10⁻⁴ Pa. The rate of evaporation for LiF is 0.02 to 0.04 nm/s and the rate of evaporation for other materials is 0.3 to 0.4 nm/s. All the steps of vapor deposition were carried out in a vacuum.

### [Example 13]

The procedure in Example 12 was repeated to prepare an OLED element except that the charge transporting varnish was replaced by the one prepared in Example 10.

### [Example 14]

The procedure in Example 12 was repeated to prepare an OLED element except that the charge transporting varnish was replaced by the one prepared in Example 11.

### [Comparative Example 7]

The procedure in Example 12 was repeated to prepare an OLED element except that the charge transporting varnish was replaced by the one prepared in Comparative Example 7.

The OLED elements prepared in Examples 12 to 14 and Comparative Example 7 were tested for characteristic properties. The characteristic properties, Ip, and conductivity are shown in Table 6.

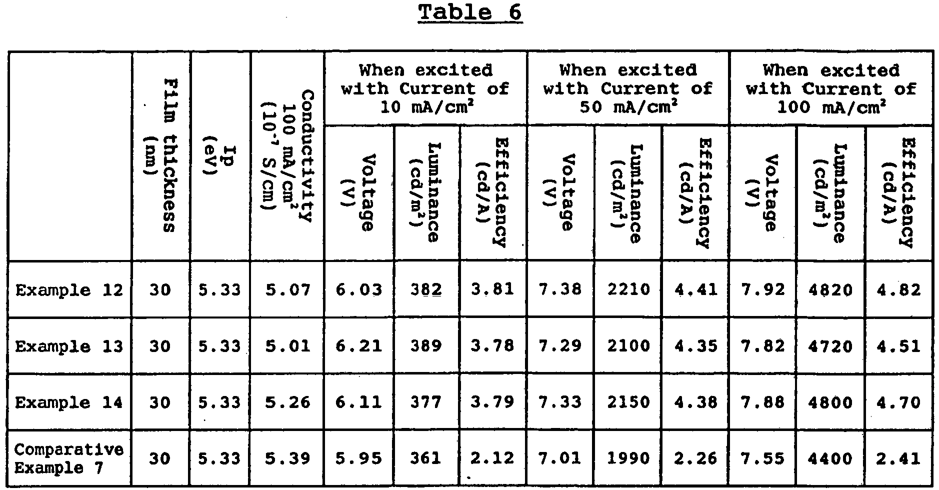

The characteristic properties of the OLED elements were measured by using an apparatus for measuring the light-emitting efficiency of organic EL elements (Model EL1003, from PRECISE GAUGE Co., Ltd.). They are indicated in terms of voltage, luminance, and light-emitting efficiency which are measured at a voltage to start light emission or when current exceeds 10 mA/cm², 50 mA/cm², or 100 mA/cm².

Conductivity was calculated from the current-voltage characteristics which were observed when a specimen prepared as follows was excited with a current of 100 mA/cm² for a film thickness of 30 nm. The specimen was prepared by forming a hole transporting thin film on an ITO-coated glass substrate and then depositing aluminum (100 nm thick) on the thin film in a vacuum deposition apparatus. Incidentally, the film thickness was measured by using a surface configuration measuring apparatus (Model DEKTAK3ST, from ULVAC, Inc.) and Ip was measured by using a photoelectron spectrometer (Model AC-2, from RIKEN KEIKI Co., Ltd.).

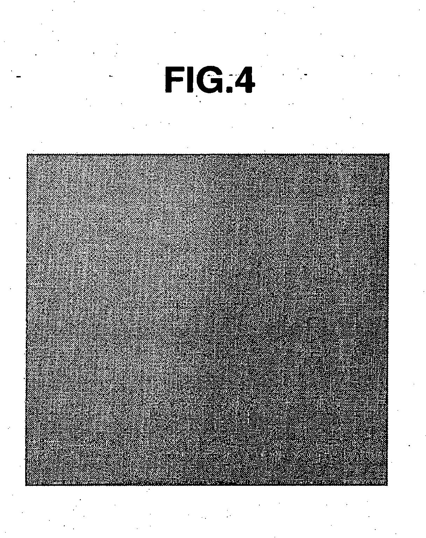

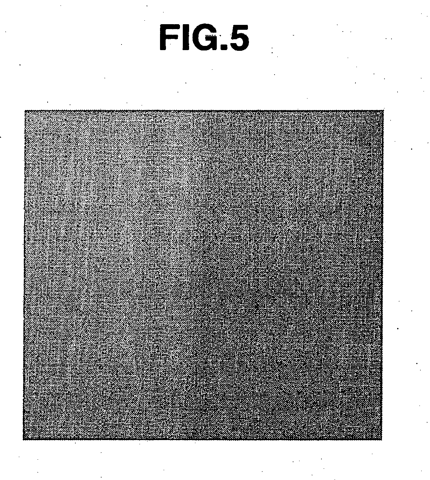

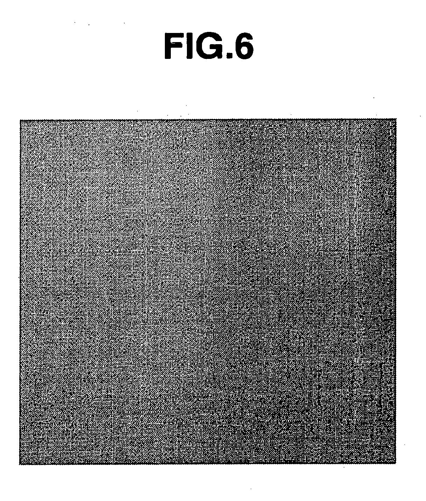

Figs. 4 to 7 show respectively the light emitting surfaces of the OLED elements (driven at 8V) which were prepared in Examples 12 to 14 and Comparative Example 7. Incidentally, the light emitting surface was observed and photographed by using an optical microscope × 10 (Model ECLIPSE ME600, from NIKON CORPORATION).

It is noted from Table 6 that the OLED element in Comparative Example 7 is lower in driving voltage as well as light emission efficiency at current of 10, 50, and 100 mA/cm² than the OLED elements in Examples 12 to 14. The OLED element in Comparative Example 7 has a charge transporting thin film as a hole injection layer which is formed from PTA containing a large amount of its oxidized form, whereas the OLED elements in Examples 12 to 14 each has a charge transporting thin film as a hole injection layer which is formed from PTA containing a small amount of its oxidized form. The low driving voltage is desirable but the low light emission efficiency is undesirable for the organic EL elements.

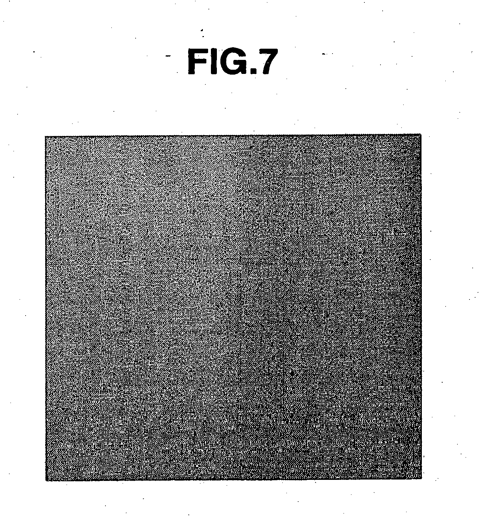

The reason why the OLED elements have a low light emission efficiency despite their low driving voltage will be obvious if Figs. 4 to 7 are compared and examined carefully. The OLED elements prepared in Examples 12 to 14 have a uniform light emitting surface as shown in Figs. 4 to 6. By contrast, the OLED element prepared in Comparative Example 7 has an uneven light emitting surface with dark spots and bright spots as shown in Fig. 7. It is considered that the OLED element in Comparative Example 7 has a low driving voltage owing to dark spots and bright spots at which charges concentrate, but it has a low light emission efficiency owing to uneven light emission in the surface. Uneven parts such as dark spots and bright spots that occur in electroluminescence are presumably associated with the surface roughness of the hole injection layer. Such uneven parts bring about electric short circuits in organic EL elements and result in uneven light emission; therefore, they are detrimental to the efficient economical production of organic EL elements with an expanded process margin.

Incidentally, it is apparent from Example 14 that a satisfactory OLED element can be produced as in Example 12 that employs PTA originally containing a less amount of its oxidized form from PTA containing a large amount of its oxidized form if it is purified by the method for purification according to the present invention.

## Claims

1. A method of purification of an oligoaniline compound which comprises the steps of:
dissolving an oligoaniline compound represented by the formula (1) below in a solvent, thereby giving a solution containing the oligoaniline compound, the oligoaniline compound
- being crude oligoaniline compound as produced, or
- containing oligoaniline in oxidised form and having an absorption coefficient ε at a wavelength of 560 nm of from 80 to 1000;
treating the solution with activated carbon in an amount of 4 to 20 wt% based on the amount of the oligoaniline compound;
removing the activated carbon, and
performing recrystallisation,
thereby obtaining a purified oligoaniline compound represented by formula (1) which has an absorption coefficient ε at a wavelength of 560 nm no higher than 30: 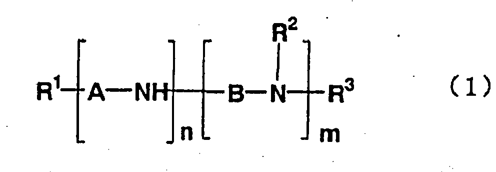 where
R¹, R², and R³ independently denote hydrogen, hydroxyl group, halogen group, amino group, silanol group, thiol group, carboxyl group, sulfonic group, phosphoric group, phosphate group, ester group, thioester group, amide group, nitro group, monovalent hydrocarbon group, organooxy group, organoamino group, organosilyl group, organothio group, acyl group or sulfonyl group; and A and B independently denote divalent groups of formula (2) or (3) below: 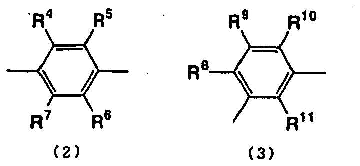
where R⁴ to R¹¹ independently denote hydrogen, hydroxyl group, halogen group, amino group, silanol group, thiol group, carboxyl group, sulfonic group, phosphoric group, phosphate group, ester group, thioester group, amide group, nitro group, monovalent hydrocarbon group, organooxy group, organoamino group, organosilyl group, organothio group, acyl group or sulfonyl group;
m and n independently denote integers equal to or larger than 1 such that m + n ≤ 20.

2. A method as defined in claim 1 wherein the purified oligoaniline compound represented by the formula (1) contains no more than 1 ppm of residual metals including Li, Mg, Ca, Fe, Cu, Zn, Ti, Sn, Na and K.

3. A method as defined in claim 1 or 2 wherein said oligoaniline compound is one represented by the formula (4) below: 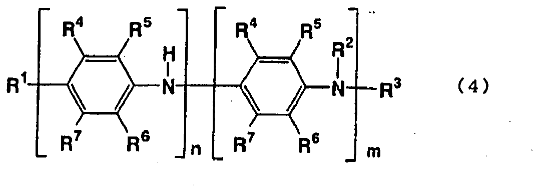 where R¹ to R⁷, m and n are as defined above.

4. A method as defined in any one of claims 1 to 3 in which in formula (1) R¹ denotes hydrogen and R³ denotes phenyl group.

5. A method according to any one of claims 1 to 4 in which solvent used for the dissolution and recrystallisation of the oligoaniline compound is selected from 1,4-dioxane, tetrahydrofuran, 1,3-dioxolane, diethylene glycol diethyl ether and acetonitrile.

6. A method of manufacture of an oligoaniline compound, the compound being of formula (1) as defined in claim 1, comprising
synthesising the compound to obtain it in crude form, and
purifying the crude compound, optionally after storage, using a purification procedure in accordance with a method of any one of claims 1 to 5.

7. A method comprising
producing a purified oligoaniline compound, by a method according to any one of claims 1 to 6;
making a charge-transporting varnish containing the purified oligoaniline compound.

8. A method according to claim 7, further comprising forming a charge-transporting thin film of the varnish, the film having an average surface roughness no larger than 1nm.

9. A method according to claim 8 comprising making an organic electroluminescence element including said charge-transporting thin film.

10. An oligoaniline compound represented by the formula (1) below which contains no more than 1 ppm of residual metals including Li, Mg, Ca, Fe, Cu, Zn, Ti, Sn, Na, and K: 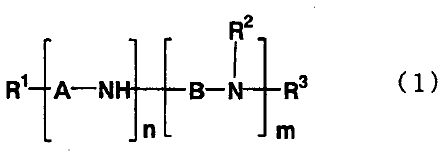 where R¹ denotes hydrogen, R² denotes hydrogen, hydroxyl group, halogen group, amino group, silanol group, thiol group, carboxyl group, sulfonic group, phosphoric group, phosphate group, ester group, thioester group, amide group, nitro group, monovalent hydrocarbon group, organooxy group, organoamino group, organosilyl group, organothio group, acyl group, or sulfonyl group, R³ denotes phenyl group and A and B independently denote a divalent group of formula (2) or (3) below. 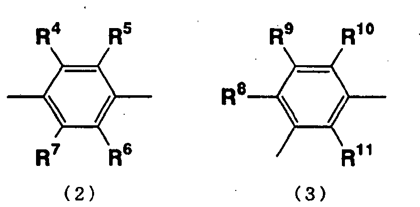 where R⁴ to R¹¹ independently denote hydrogen, hydroxyl group, halogen group, amino group, silanol group, thiol group, carboxyl group, sulfonic group, phosphoric group, phosphate group, ester group, thioester group, amide group, nitro group, monovalent hydrocarbon group, organooxy group, organoamino group, organosilyl group, organothio group, acyl group, or sulfonyl group; and m and n independently denote integers equal to or larger than 1 such that m + n s 20.

11. An oligoaniline compound of claim 10 which has an absorption coefficient ε no higher than 400 at a wavelength of 560 nm.

12. An oligoaniline compound as defined in claim 10 or 11, which is represented by the formula (4) below. 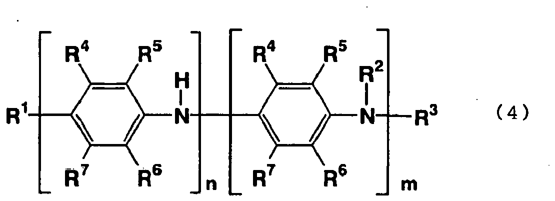 where R¹ to R⁷ and m and n are as defined in claim 10.

13. A charge transporting varnish which contains an oligoaniline compound as defined in any of claims 10 to 12.

14. A charge transporting thin film prepared from the charge transporting varnish defined in claim 13, which is **characterized by** an average value of surface roughness no larger than 1 nm. a

15. An organic electroluminescence element which has a charge transporting thin film as defined in claim 14.

## Patentansprüche

1. Verfahren zur Reinigung einer Oligoanilinverbindung, das folgende Schritte umfasst:
Lösen einer Oligoanilinverbindung, die durch die nachstehende Formel (1) dargestellt ist, in einem Lösungsmittel, wodurch eine Lösung erhalten wird, welche die Oligoanilinverbindung enthält, wobei die Oligoanilinverbindung
- eine rohe Oligoanilinverbindung ist, wie sie hergestellt wurde, oder
- ein Oligoanilin in oxidierter Form enthält und einen Adsorptionskoeffizienten ε bei einer Wellenlänge von 560 nm von 80 bis 1000 aufweist;
Versetzen der Lösung mit Aktivkohle in einer Menge von 4 bis 20 Gew.-%, basierend auf der Menge der Oligoanilinverbindung;
Entfernen der Aktivkohle; und
Durchführen von Umkristallisation;
wodurch eine gereinigte Oligoanilinverbindung erhalten wird, die durch die Formel (1) dargestellt ist und einen Adsorptionskoeffizienten ε bei einer Wellenlänge von 560 nm von nicht mehr als 30 aufweist: 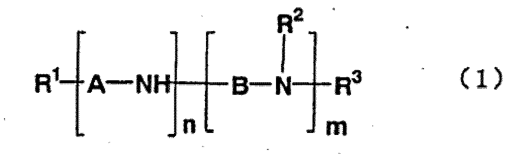 worin
R¹, R² und R³ unabhängig voneinander für Wasserstoff, eine Hydroxylgruppe, Halogengruppe, Aminogruppe, Silanolgruppe, Thiolgruppe, Carboxylgruppe, Sulfonsäuregruppe, Phosphorsäuregruppe, Phosphatgruppe, Estergruppe, Thioestergruppe, Amidgruppe, Nitrogruppe, einwertige Kohlenwasserstoffgruppe, Organooxygruppe, Organoaminogruppe, Organosilylgruppe, Organothiogruppe, Acylgruppe oder Sulfonylgruppe stehen; und A und B unabhängig voneinander für zweiwertige Gruppen der nachstehenden Formel (2) oder (3) stehen: 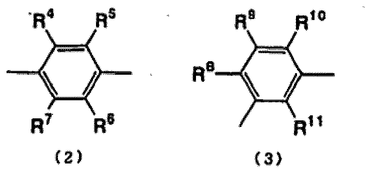 worin R⁴ bis R¹¹ unabhängig voneinander für Wasserstoff, eine Hydroxylgruppe, Halogengruppe, Aminogruppe, Silanolgruppe, Thiolgruppe, Carboxylgruppe, Sulfonsäuregruppe, Phosphorsäuregruppe, Phosphatgruppe, Estergruppe, Thioestergruppe, Amidgruppe, Nitrogruppe, einwertige Kohlenwasserstoffgruppe, Organooxygruppe, Organoaminogruppe, Organosilylgruppe, Organothiogruppe, Acylgruppe oder Sulfonylgruppe stehen;
m und n unabhängig für ganze Zahlen gleich oder größer als 1 stehen, sodass gilt: m + n ≤ 20.

2. Verfahren nach Anspruch 1, wobei die durch die Formel (1) dargestellte gereinigte Oligoanilinverbindung nicht mehr als 1 ppm Restmetalle, einschließlich Li, Mg, Ca, Fe, Cu, Zn, Ti, Sn, Na und K enthält.

3. Verfahren nach Anspruch 1 oder 2, wobei die Oligoanilinverbindung eine ist, die durch die nachstehende Formel (4) dargestellt ist: 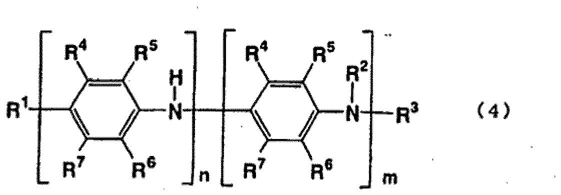 worin R¹ bis R⁷, m und n wie oben definiert sind.

4. Verfahren nach einem der Ansprüche 1 bis 3, wobei in Formel (1) R¹ für Wasserstoff steht und R³ für eine Phenylgruppe steht.

5. Verfahren nach einem der Ansprüche 1 bis 4, wobei das zum Lösen und zur Umkristallisation der Oligoanilinverbindung verwendete Lösungsmittel aus 1,4-Dioxan, Tetrahydrofuran, 1,3-Dioxolan, Diethylenglykoldiethylether und Acetonitril ausgewählt ist.

6. Verfahren zur Herstellung einer Oligoanilinverbindung, wobei die Verbindung der Formel (1) wie in Anspruch 1 definiert entspricht, das Folgendes umfasst:
Synthetisieren der Verbindung, um sie in roher Form zu erhalten, und
Reinigen der rohen Verbindung, gegebenenfalls nach Lagerung, unter Anwendung eines Reinigungsverfahrens gemäß einem Verfahren nach einem der Ansprüche 1 bis 5.

7. Verfahren, das Folgendes umfasst:
Herstellen einer gereinigten Oligoanilinverbindung durch ein Verfahren nach einem der Ansprüche 1 bis 6;
Herstellen einer ladungstransportierenden Lacks, der die gereinigte Oligoanilinverbindung enthält.

8. Verfahren nach Anspruch 7, das weiters Bilden eines ladungstransportierenden Dünnfilms aus dem Lack umfasst, wobei der Film eine mittlere Oberflächenrauigkeit von nicht mehr als 1 nm aufweist.

9. Verfahren nach Anspruch 8, das die Herstellung eines organischen Elektrolumineszenzelements umfasst, das den ladungstransportierenden Dünnfilm umfasst.

10. Oligoanilinverbindung, die durch die nachstehende Formel (1) dargestellt ist und nicht mehr als 1 ppm Restmetalle, einschließlich Li, Mg, Ca, Fe, Cu, Zn, Ti, Sn, Na und K umfasst: 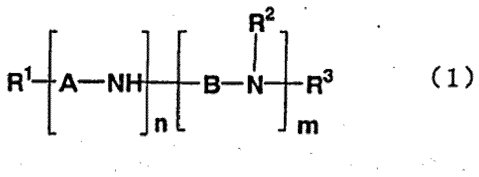 worin
R¹ für Wasserstoff steht, R² für Wasserstoff, eine Hydroxylgruppe, Halogengruppe, Aminogruppe, Silanolgruppe, Thiolgruppe, Carboxylgruppe, Sulfonsäuregruppe, Phosphorsäuregruppe, Phosphatgruppe, Estergruppe, Thioestergruppe, Amidgruppe, Nitrogruppe, einwertige Kohlenwasserstoffgruppe, Organooxygruppe, Organoaminogruppe, Organosilylgruppe, Organothiogruppe, Acylgruppe oder Sulfonylgruppe steht, R³ für eine Phenylgruppe steht und A und B unabhängig voneinander für zweiwertige Gruppen der nachstehenden Formel (2) oder (3) stehen: 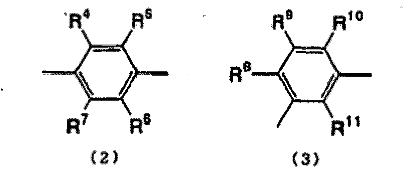 worin R⁴ bis R¹¹ unabhängig voneinander für Wasserstoff, eine Hydroxylgruppe, Halogengruppe, Aminogruppe, Silanolgruppe, Thiolgruppe, Carboxylgruppe, Sulfonsäuregruppe, Phosphorsäuregruppe, Phosphatgruppe, Estergruppe, Thioestergruppe, Amidgruppe, Nitrogruppe, einwertige Kohlenwasserstoffgruppe, Organooxygruppe, Organoaminogruppe, Organosilylgruppe, Organothiogruppe, Acylgruppe oder Sulfonylgruppe stehen; und m und n unabhängig für ganze Zahlen gleich oder größer als 1 stehen, sodass gilt: m + n ≤ 20.

11. Oligoanilinverbindung nach Anspruch 10, die einen Adsorptionskoeffizienten ε von nicht mehr als 400 bei einer Wellenlänge von 560 nm aufweist.

12. Oligoanilinverbindung nach Anspruch 10 oder 11, die durch die nachstehende Formel (4) dargestellt ist: 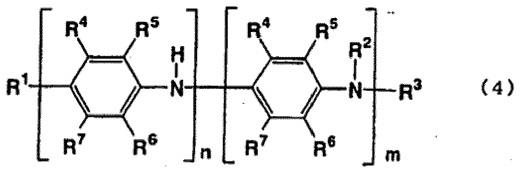 worin R¹ bis R⁷, m und n wie in Anspruch 10 definiert sind.

13. Ladungstransportierender Lack, der eine Oligoanilinverbindung nach einem der Ansprüche 10 bis 12 enthält.

14. Ladungstransportierender Dünnfilm, der aus einem ladungstransportierenden Lack nach Anspruch 13 hergestellt ist und durch einen mittleren Oberflächenrauigkeitswert von nicht mehr als 1 nm gekennzeichnet ist.

15. Organisches Elektrolumineszenzelement, das einen ladungstransportierenden Dünnfilm nach Anspruch 14 umfasst.

## Revendications

1. Procédé de purification d'un composé oligo-aniline, qui comprend les étapes consistant à :
dissoudre un composé oligo-aniline représenté par la formule (1) ci-dessous dans un solvant, en obtenant ainsi une solution contenant le composé oligo-aniline, le composé oligoaniline
- étant un composé oligo-aniline brut tel que produit, ou
- contenant une oligo-aniline sous forme oxydée et ayant un coefficient d'absorption ε à une longueur d'onde de 560 nm de 80 à 1000 ;
traiter la solution avec du charbon activé en une quantité de 4 à 20 % en poids par rapport à la quantité du composé oligo-aniline ;
retirer le charbon activé, et
effectuer une recristallisation,
en obtenant ainsi un composé oligo-aniline purifié représenté par la formule (1) qui a un coefficient d'absorption ε à une longueur d'onde de 560 nm ne dépassant pas 30 : 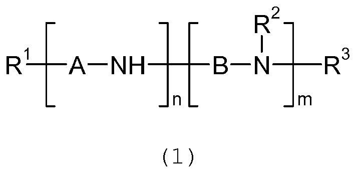 où
R¹, R² et R³ désignent indépendamment l'hydrogène, un groupe hydroxyle, un groupe halogéno, un groupe amino, un groupe silanol, un groupe thiol, un groupe carboxyle, un groupe sulfonique, un groupe phosphorique, un groupe phosphate, un groupe ester, un groupe thioester, un groupe amide, un groupe nitro, un groupe hydrocarboné monovalent, un groupe organoxy, un groupe amino organique, un groupe silyle organique, un groupe thio organique, un groupe acyle ou un groupe sulfonyle ;
et A et B désignent indépendamment des groupes divalents de formule (2) ou (3) ci-dessous : 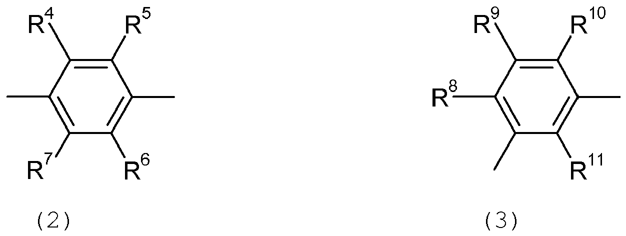
où R⁴ à R¹¹ désignent indépendamment l'hydrogène, un groupe hydroxyle, un groupe halogéno, un groupe amino, un groupe silanol, un groupe thiol, un groupe carboxyle, un groupe sulfonique, un groupe phosphorique, un groupe phosphate, un groupe ester, un groupe thioester, un groupe amide, un groupe nitro, un groupe hydrocarboné monovalent, un groupe organoxy, un groupe amino organique, un groupe silyle organique, un groupe thio organique, un groupe acyle ou un groupe sulfonyle ; et m et n désignent indépendamment des entiers égaux ou supérieurs à 1, tels que m + n ≤ 20.

2. Procédé selon la revendication 1, dans lequel le composé oligo-aniline purifié représenté par la formule (1) contient au plus 1 ppm de métaux résiduels comprenant Li, Mg, Ca, Fe, Cu, Zn, Ti, Sn, Na et K.

3. Procédé selon la revendication 1 ou 2, dans lequel ledit composé oligo-aniline est représenté par la formule (4) ci-dessous : 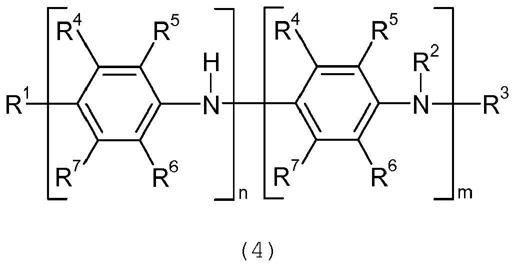 où R¹ à R⁷, m et n sont tels que définis ci-dessus.

4. Procédé selon l'une quelconque des revendications 1 à 3, dans lequel, dans la formule (1), R¹ désigne l'hydrogène et R³ désigne un groupe phényle.

5. Procédé selon l'une quelconque des revendications 1 à 4, dans lequel le solvant utilisé pour la dissolution et la recristallisation du composé oligo-aniline est choisi parmi le 1,4-dioxane, le tétrahydrofurane, le 1,3-dioxolane, l'éther diéthylique de diéthylèneglycol et l'acétonitrile.

6. Procédé de fabrication d'un composé oligo-aniline, le composé étant de formule (1) comme défini dans la revendication 1, comprenant
la synthèse du composé pour qu'il soit obtenu sous forme brute, et
la purification du composé brut, éventuellement après stockage, par utilisation d'une procédure de purification conformément à un procédé selon l'une quelconque des revendications 1 à 5.

7. Procédé comprenant
la production d'un composé oligo-aniline purifié par un procédé selon l'une quelconque des revendications 1 à 6 ;
la réalisation d'un vernis de transport de charges contenant le composé oligo-aniline purifié.

8. Procédé selon la revendication 7, comprenant en outre la formation d'un film mince de transport de charges du vernis, le film ayant une rugosité de surface moyenne ne dépassant pas 1 nm.

9. Procédé selon la revendication 8, comprenant la réalisation d'un élément électroluminescent organique contenant ledit film mince de transport de charges.

10. Composé oligo-aniline représenté par la formule (1) ci-dessous qui contient au plus 1 ppm de métaux résiduels comprenant Li, Mg, Ca, Fe, Cu, Zn, Ti, Sn, Na, et K : 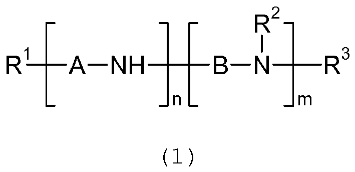 où R¹ désigne l'hydrogène, R² désigne l'hydrogène, un groupe hydroxyle, un groupe halogéno, un groupe amino, un groupe silanol, un groupe thiol, un groupe carboxyle, un groupe sulfonique, un groupe phosphorique, un groupe phosphate, un groupe ester, un groupe thioester, un groupe amide, un groupe nitro, un groupe hydrocarboné monovalent, un groupe organoxy, un groupe amino organique, un groupe silyle organique, un groupe thio organique, un groupe acyle ou un groupe sulfonyle, R³ désigne un groupe phényle, et A et B désignent indépendamment des groupes divalents de formule (2) ou (3) ci-dessous : 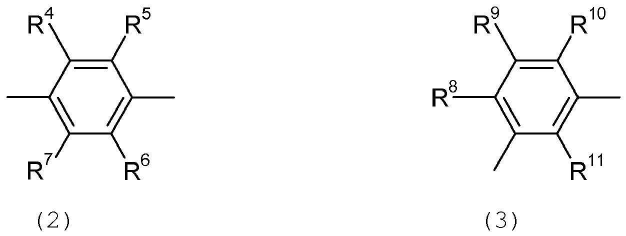 où R⁴ à R¹¹ désignent indépendamment l'hydrogène, un groupe hydroxyle, un groupe halogéno, un groupe amino, un groupe silanol, un groupe thiol, un groupe carboxyle, un groupe sulfonique, un groupe phosphorique, un groupe phosphate, un groupe ester, un groupe thioester, un groupe amide, un groupe nitro, un groupe hydrocarboné monovalent, un groupe organoxy, un groupe amino organique, un groupe silyle organique, un groupe thio organique, un groupe acyle ou un groupe sulfonyle ; et m et n désignent indépendamment des entiers égaux ou supérieurs à 1, tels que m + n ≤ 20.

11. Composé oligo-aniline selon la revendication 10, qui a un coefficient d'absorption ε ne dépassant pas 400 à une longueur d'onde de 560 nm.

12. Composé oligo-aniline selon la revendication 10 ou 11, qui est représenté par la formule (4) ci-dessous : 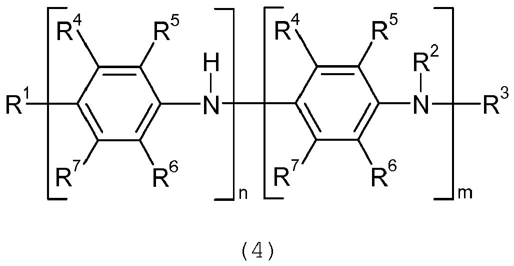 où R¹ à R⁷, m et n sont tels que définis dans la revendication 10.

13. Vernis de transport de charges qui contient un composé oligo-aniline tel que défini dans l'une quelconque des revendications 10 à 12.

14. Film mince de transport de charges préparé à partir du vernis de transport de charges défini dans la revendication 13, qui est **caractérisé par** une valeur moyenne de rugosité de surface ne dépassant pas 1 nm.

15. Elément électroluminescent organique qui a un film mince de transport de charges tel que défini dans la revendication 14.
